# EUROPEAN PATENT APPLICATION

(11) **EP 1 842 497 A1**
(43) Date of publication of application: **10.10.2007**
(21) Application number: 07105569.3
(22) Date of filing: 03.04.2007
(51) Int. Cl.: A61B 17/16, A61C 3/02, A61C 8/00

(54) **Identification system of the bone type**

(30) Priority: 04.04.2006 IT MI20060653
(71) Applicant: I.D.I. Evolution S.r.l., 20052 Monza MI (IT)
(72) Inventor: Piantoni, Angiolino, 20050, LESMO (Milan) (IT)
(74) Representative: Martegani, Franco

(57) **Abstract**

A cutter of a drill for surgical applications comprises a shank having a constraining mechanism to the drill (2) at an upper end and a head suitable for operating on the surface to be intervened. Said head comprises a tip (5) at the lower end and an opposite pair of longitudinal grooves positioned for substantially the whole length of the body (4) with a broadened central portion on which the depth stops can be assembled.

## Description

In surgical procedures for the insertion of an implant, the pre-operative definition of the bone type referring to its density according to a universally acknowledged classification (Mish classification) allows a more accurate programming of the type of intervention, the selection of the device and rehabilitation prognosis.

The techniques currently available for defining the bone density are CT (Computed Tomography) and bioptic tests.

CT is a non-invasive pre-operative test which allows high-quality images to be obtained, with last-generation tomographs, with extremely low exposures. The result is a sectional radiogram of the area in which the insertion of the device is envisaged, on which it is possible to measure the bone density by measuring the intensity of the grey value (which increases with an increase in the bone density) according to a predefined scale (HOUNSFIELD scale).

This measurement with last-generation machines is extremely reliable but it can only be effected in specialized centres and there are not always indications for requesting it.

The Hounsfield scale of the bone density comprises about 2000 values indicating the grey levels available in a CT system; the lowest is -1000 and corresponds to air, 0 is water and + 1000 is the maximum bone density.

According to the document PIERAZZINI A: computed tomography of the maxillofacial section - 1996 published by UTET., the bone D1 has values > 1250 Hounsfield units (HU)., the bone D2 from 850 HU to 1250 HU, the bone D3 from 350 to 850 HU and the bone D4 < 350(HU).

The other technique is intra-operative and invasive and the results cannot be provided within a short period of time (for being useful during the intervention) as the survey techniques are long and laborious (in some cases weeks).

It consists in removing a core of 8-10 mm in the area to be used for the implant insertion, a histological test is effected and the mineralization degree of the sample is evaluated. A soft bone has a mineralization degree of up to 20%, a medium bone from 20% to 80% and a hard bone over 80%. Alternatively a scanning can be effected with a Scanco medical AG: mcirotac system to digitally obtain the same result.

The repeatable intra-operative instrumental definition of the bone density remains a problem which has yet to be solved.

A market analysis shows that some companies are already moving in this direction but with empirical methods which are too dependent on the efficacy of single individuals and the state of wear of the instruments.

The object of the present invention relates to an identification system of the bone type based on a cutter, a mechanical reader and a surgical motor.

The cutter, made of steel in a single piece to avoid the unwelcome or dangerous disconnection of the parts, has a defined diameter and an adjustable stop which prevents in-depth overpreparations.

The reader has a screw morphology of 2.5 mm in diameter with 3 longitudinal discharges along the whole length.

The surgical motor has an electronic section which controls the rotation rate, rotation force, number of revs effected and electric absorption to keep the rotation force and velocity constant, also inserting the pitch value of the implant and possible indication of amount of millimeters our device has penetrated in the preparation.

The depth stop can be electronically regulated.

The control of these parameters allows the motor to provide the utilization indications on the rotation force expressed in Newton, which when processed become:
- average screwing force (the average of the measurements effected with time)
- screwing peak force (the maximum screwing force)
- integral of the stress-millimeters function
- function trend graph.

The process for measuring these parameters is effected as follows:
a hole is made with the cutter as far as the preestablished stop. During this operation both the sensitivity of the operator and the wear of the instrument influence the quality of the reading (it will be indicated on the maintenance handbook that in order to fully perform, the rotating instrument must be substituted after 25 uses, for example).

The surgical motor is prepared for the reading procedure which envisages a rotation rate of (x N from 10 to 40 to be defined) and a rotation rate of 35 revs per minute approximately (rate to be defined).

The low velocity combined with the high operating torque make the procedure non-brutal and perfectly controllable on the part of the operator. Furthermore, the particular design of the reader ensures that the operator does not have to exert any force and the reader will proceed in the alveolus as far as the preestablished depth, automatically interrupting the rotation.

With this procedure, the components relating to the influence of the operator are annulled allowing the reading on the part of the instrument to be repeatable and standardized.

The data obtained from the surgical motor indicate the quantity of energy necessary for maintaining the rotation rate constant which is proportional to the resistance encountered by the reader for proceeding to the alveolus prepared.

As the alveolus has constant dimensions, the forces and rates in question are constant and the operator has no influence, the values registered can be related to the bone hardness whose reading is effected (the greater the resistance registered, the greater the mineralization degree of the bone tissue will be).

In order to have scientific validity, theses values are compared with histological data to enable the resistance values registered to be accurately assigned to the hardness degree of the bone according to international classifications.

The data which can be acquired from the machine can be processed in various forms:
Average value: the average of the resistive force values registered for each fraction of a millimeter.
Peak value: the maximum resistance value reached during the reading.
Integral of the force/depth function: this value gives a more accurate idea of the contact which develops between the reader and the bone in which it is engaged.

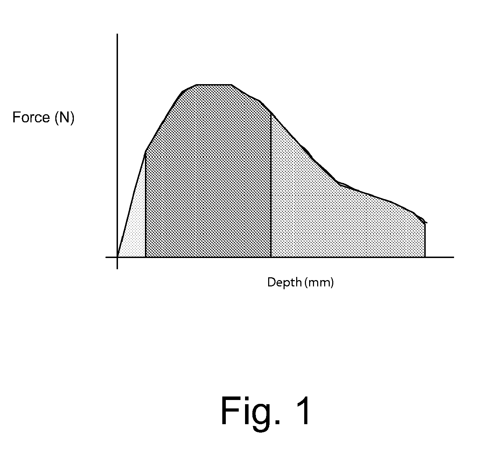

The graph of figure 1 illustrates the graphic indication which appears on the monitor of the surgical motor, it helps the operator to understand if there are non-homogeneous resistance areas and not to avail of the average values alone.

The different shades of grey show various resistance areas. This allows a more precise interpretation of the data registered and which can be interpreted by the clinician.

An inter-operative knowledge of these data can modify the doctor's approach with respect to numerous aspects of the treatment, from the selection of the device, its post-operative management and prognosis; the behaviour towards the different types of bone, in fact, changes significantly and the sensitivity of the operator is not a sensitive value (scientifically demonstrated).

The present invention relates to an identification system of the bone type based on a cutter of a drill for surgical applications, with a reader and a surgical motor capable of interpreting the resistance values, converting them into data relating to the bone densitometry.

An objective of the present invention is to avoid the non-repeatability of bone resistance values which can be defined with high-velocity rotating instruments or the sensitivity of the operator as described amply above, but to provide an objective, repeatable scientific instrument which can be used intraoperatively to identify the type of bone with which it is engaged.

These and other objectives are achieved by the present invention by providing a cutter for drills for surgical applications, according to claim 1, to which reference should be made for the sake of brevity, a reader according to claim 2, to which reference should be made for the sake of brevity and a motor for surgical applications according to claim 3 to which reference should be made for the sake of brevity.

Further characteristics of the invention are defined in the other claims enclosed with the present patent application.

Further objectives and advantages of the present invention will appear evident from the following description and enclosed drawings, provided for purely illustrative and non-limiting purposes, in which:
- figure 1 is a graph of the trend of the forces measured in relation to the depth by the system according to the present invention;

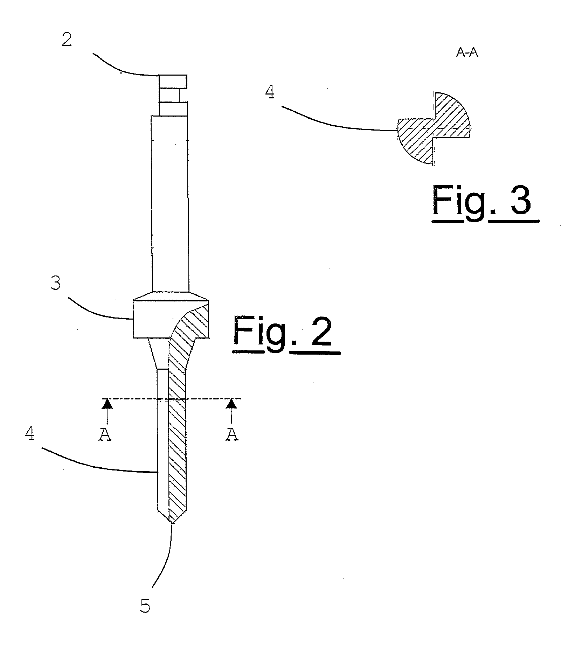

- figure 2 represents a front view of a cutter according to the present invention;
- figure 3 represents a view along the section A-A of the cutter according to the present invention;
- figure 4 represents a front view of a reader according to the present invention;

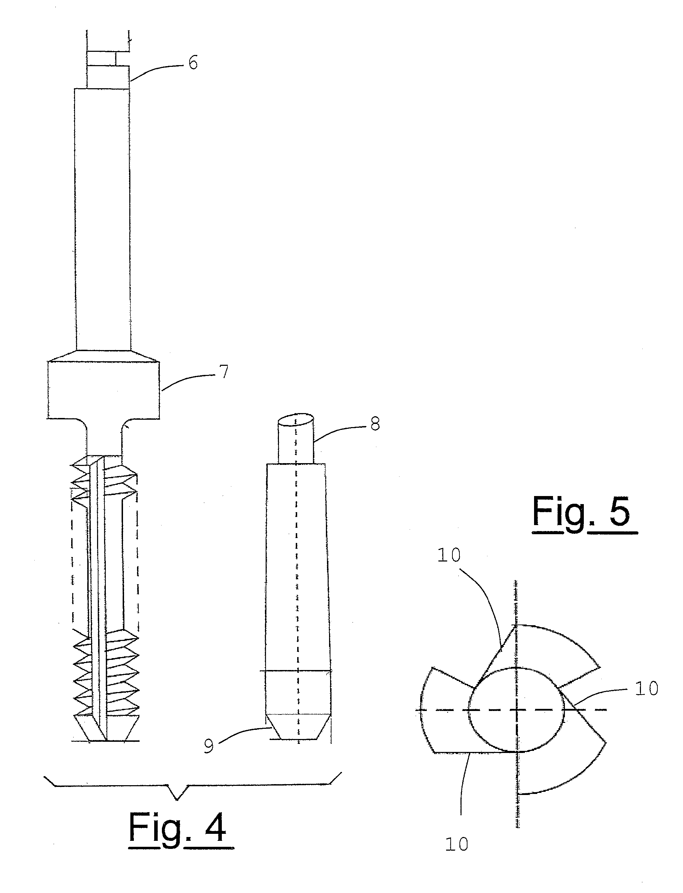

- figure 5 represents a view along the section A-A of the reader according to the present invention.

The cutter and the reader have a portion for fixing in the counterangles for surgical use according to the standard UNI EN ISO 1792-1.

With particular reference to the above figures, the cutter according to the present invention comprises a constraining mechanism 2, according to the regulation UNI EN ISO 1797-1, for maniples of surgical motors.

At the opposite end of the constraining mechanism there is a separation ring 3 of the upper part and the underlying operative part or head which comes into contact with the surface to be operated, on which mechanical depth stops can be assembled.

This operative part of the cutter comprises a body 4 which has a tip 5 at its end. Both the head and the body have an opposite pair of longitudinal grooves (section A-A) arranged for substantially the whole length of the tip, preferably both having right-angle sections.

In order to guarantee resistance and duration of the cut, the cutter is made of medical steel.

The characteristics of the cutter of a drill for surgical applications, object of the present invention are evident from the above description.

With particular reference to the above figures, the reader according to the present invention comprises a constraining mechanism 6, according to the regulation UNI EN ISO 1797-1, for the maniples of the surgical motors.

At the opposite end of the constraint there is a separation ring 7 from the supporting part and the underlying operative part or reader which comes into contact with the surface to be intervened.

This operative part comprises a portion 8 of 14 mm produced with the morphology of a screw having a diameter at the tip 9 of 2.8 mm and terminating with a diameter of 2.5 mm.

The operating part is also provided with three vertical grooves 10 for the whole length (section A-A).

The surgical motor must have the specific characteristic of being able to convert the data relating to the resistance encountered by the reader during its descent into the implant alveolus into data such as the average value, peak value, integral function of the force/millimeter curve and operating depth. These values will correspond to International bone-type classifications.

Finally, numerous variants can obviously be applied to the cutter, object of the present invention, and to the reader, all included in the novelty principles inherent in the inventive concept.

In the practical embodiment of the invention, the materials, forms and dimensions of the details illustrated can vary according to the requirements and can be substituted with other technically equivalent elements.

The scope of the invention is defined in the claims enclosed with the present patent application.

## Claims

1. A cutter of a drill for surgical applications comprises a shank having a constraining mechanism to the drill (2) at an upper end and a head suitable for operating on the surface to be intervened, **characterized in that** said head comprises a tip (5) at the lower end and an opposite pair of longitudinal grooves positioned for substantially the whole length of the body (4) with a broadened central portion on which the depth stops can be assembled.

2. The drill cutter according to claim 1, **characterized in that** said longitudinal grooves both have right-angle sections.

3. The drill cutter according to claim 1, **characterized in that** said constraining mechanism is produced according to the regulation UNI EN ISO 1797-1.

4. The drill cutter according to claim 1, **characterized in that** the material of which it is made is medical steel.

5. The drill cutter according to claim 1, having a ring on which the depth stops can be wedge-inserted.

6. A reader for a drill according to claim 1, **characterized in that** it comprises in a single piece, the shank for fixing a leg with the surgical maniple, a stop produced with an increase in diameter and an operating portion of about 16 mm produced with the morphology of a metal tapper with a diameter of about 2.8 mm at the apex (9) and about 2.5 mm towards the leg (8), a pitch of about 0.75 and 3 longitudinal cavities which allow the tapping cuttings to be discharged.

7. A reader for a drill according to claim 1, **characterized in that** the material of which it is made is medical steel.

8. A surgical motor **characterized in that** it must analyze and process the resistance values encountered by the cutter produced according to claim 1, which reads the consistency proceeding at a low velocity in the alveolus created by the first cutter.
